Europäisches Patentamt

European Patent Office

Office européen des brevets

(19)

(11) Veröffentlichungsnummer: **0 241 710 B1**

(12) **EUROPÄISCHE PATENTSCHRIFT**

(45) Veröffentlichungstag der Patentschrift: **24.06.92**

(21) Anmeldenummer: **87103517.6**

(22) Anmeldetag: **11.03.87**

(51) Int. Cl.⁵: **A61K 31/715**, A61K 49/04, A61K 9/14, A23L 1/05

Die Akte enthält technische Angaben, die nach dem Eingang der Anmeldung eingereicht wurden und die nicht in dieser Patentschrift enthalten sind.

(54) **Guar-Mehl.**

(30) Priorität: **18.04.86 DE 3613219**

(43) Veröffentlichungstag der Anmeldung:
**21.10.87 Patentblatt 87/43**

(45) Bekanntmachung des Hinweises auf die Patenterteilung:
**24.06.92 Patentblatt 92/26**

(84) Benannte Vertragsstaaten:
**AT BE CH DE ES FR GB GR IT LI LU NL SE**

(56) Entgegenhaltungen:
**EP-A- 0 080 673**
**GB-A- 2 030 583**

(73) Patentinhaber: **Speck, Ulrich, Dr.**
**Benediktinerstrasse 50**
**W-1000 Berlin 28(DE)**

(72) Erfinder: **Speck, Ulrich, Dr.**
**Benediktinerstrasse 50**
**W-1000 Berlin 28(DE)**
Erfinder: **Roumeliotis, Panayiotis, Dr.**
**Frankturter Strasse 12**
**W-6100 Darmstadt(DE)**

(74) Vertreter: **Wablat, Wolfgang**
**Patentanwalt Dr.-Ing. Dr. jur. Dipl.-Chem. W. Wablat Potsdamer Chaussee 47**
**W-1000 Berlin 38(DE)**

**Beschreibung**

Die Erfindung betrifft ein oral zu verabfolgendes Arzneimittel, insbesondere zur Verzögerung der Glucoseabsorption, zur Senkung des Blutcholesterinspiegels, zur Verringerung eines vorzeitigen Hungergefühls und zur Regulierung der intestinalen Peristaltik beim Menschen sowie ein oral zu verabfolgendes Kontrastmittel.

Quellmittel sind in der Regel natürlich vorkommende oder halbsynthetische quellfähige, nicht verdauliche Polysaccharide. Sie erhalten den Darminhalt geschmeidig, erhöhen dessen Viskosität und Wassergehalt. Hinzu kommt die durch die Darmfüllung gesteigerte Peristaltik. Beispiele für Quellmittel sind Methylcellulose, Carboxymethylcellulose, Agar-Agar und Guar-Mehl.

Bei allen Quellmitteln ist darauf zu achten, daß genügend Wasser aufgenommen wird, da sonst der Darminhalt verklebt und Ileusgefahr besteht.

Guar-Mehl hat die Fähigkeit, in Wasser rasch aufzuquellen. Bereits geringe Mengen an Guar-Mehl ergeben mit Wasser große Volumina Gallerte. Guar-Mehl ist ein Naturprodukt, und zwar ein Polysaccharid, welches zu etwa zwei Teilen aus Mannose und zu einem Teil aus Galaktose besteht. Guar-Mehl wird nach oraler Einnahme nicht enzymatisch hydrolysiert und daher auch nicht resorbiert. Es ist als gut verträglich bekannt.

In der EP-A-0 080 673 sind oral zu verabfolgende Guar-Mehl Tabletten beschrieben, die unter anderem aus Guar-Mehl einer Korngröße von 60 bis 500 $\mu$m hergestellt sind. Die GB-A-2 030 583 beschreibt unter Verwendung von aus Guar-Mehl hergestellte Granulate, deren
Partikel bis zu 95 Gew.-% eine Korngröße von 100 bis 1000 $\mu$m haben. Diese Granulate sind, aufgrund ihrer Partikelgröße, im Wasser leicht suspendierbar, quellen aber erst nach einigen Minuten zu einem viskosen Gel durch, so daß sie getrunken werden können.

Beide Literaturquellen weisen sowohl auf das Quellvermögen der entsprechenden Guar-Tabletten bzw. Guar-Granulate als auch auf die Verwendung von Guar als Verdickungsmittel für Appreturen und zur Senkung des Cholesterin- und Glukose-Blutspiegels hin.

In der Medizin hat Guar aus mehreren Gründen Verwendung gefunden:

a) Es verzögert die Resorption von Nährstoffen, insbesondere von Glucose, wenn es vor den Mahlzeiten eingenommen wird, wie in Lembcke,B.; Ebert,R.; Ptok,M; Caspary,W.F.; Crentzfeldt,W.; Schicha,H.; Emrich,D.; Role of gastrointestinal transit in the delay of absorption by viscous fibre (guar); Hepatogastroenterol. 31,4; 183-186 (1984) gezeigt wurde.

b) Ferner senkt es den Blutcholesteringehalt, wie in Bosello,O.; Caminacini,L.; Zocca,I.; Garbin,U.; Ferrari,F.; Davoli,A.; Effects of guar gum on plasma lipoproteins and apolipoproteins C-II and C-III in patients affected by familial combined hyperlipoproteinemia; The American Journ. of Clinical Nutrition 40, 6; 1165-1174 (1984) dargestellt wurde.

c) Es wirkt appetithemmend

d) Es reguliert die Darmperistaltik.

Insbesondere unter den Gesichtspunkten a) und b) wird Guar auch als Diätetikum und Nahrungsmittelzusatz eingesetzt.

Ein Problem bei der Verwendung von Guar als Arnzeimittel bzw. Nahrungsmittelzusatz ist die erforderliche hohe Dosis. Sowohl für die Glucoseresorptionsverzögerung als auch für die Senkung des Cholesterinspiegels sind üblicherweise Tagesdosen von mindestens 10g Guar notwendig.

Guar wird normalerweise als weißes bis gelbliches, weitgehend geschmacksneutrales Mehl zur Verfügung gestellt. Dieses Mehl kann nicht in trockenem Zustand eingenommen werden, da es Mund, Rachen und Speiseröhre verkleben würde. Mit wenig Wasser angerührt, ergibt das Mehl eine steife Gallerte, die ebenfalls nicht geschluckt werden kann. Darüber hinaus neigen die handelsüblichen Guar-Mehle zur Klumpenbildung, sofern sie nicht sehr intensiv gerührt werden. Erst beim Einrühren in eine sehr große Menge an Wasser wird eine hinreichend dünnflüssige Lösung erhalten, die getrunken werden kann.

Beim handelsüblichen Guar-Mehl besteht aufgrund des nach der Einnahme sofort einsetzenden Quellvorganges sogar eine erhebliche Erstickungsgefahr. Das naheliegende Aufschwemmen und Verdünnen des Guar-Mehls zu einer trinkbaren Lösung kommt auch nicht in Betracht, weil Guar innerhalb von Sekunden so stark quillt, daß beispielsweise zur Einnahme von 4g Guar-Mehl mindestens 400 ml Wasser erforderlich wären. Dieses Problem wurde bislang durch den Zusatz von Hilfsstoffen (Gelatine, Stärke, Puffermittel, Kieselgel) gelöst. Diese Hilfsmittel verbesserten zwar die Einnehmbarkeit des Guar-Mehls, schufen jedoch einige Probleme:

a) Die ohnehin hohe Dosis an Guar wird durch die therapeutisch unwirksamen und teilweise sogar unerwünschten Hilfsstoffe noch erhöht.

b) Die Hilfsstoffe treten in Wechselwirkung mit dem Guar-Mehl und können die erwünschte Quellbarkeit des Guar im Darm verringern. Eine vollständige Quellbarkeit des Guar ist jedoch Voraussetzung für die erwünschte pharmakologische Wirkung.

c) Die Zubereitungen erhalten grundsätzlich einen partikulären Charakter, d.h. es werden makroskopische Guar-Teilchen mit einem Durchmesser von 1 mm bis >1 cm verabreicht. Diese Teilchen müssen entweder in Wasser suspendiert oder in Tablettenform eingenommen werden.

Bedingt durch die hohe Dosis ist die täglich mehrfache Einnahme von zahlreichen oder großen Guar-Tabletten oder großen Mengen trockenen Granulats mit Wasser gerade älteren Menschen kaum zuzumuten. Bei allen partikelhaltigen Medien kommt hinzu, daß sich angequollene Teile im Mund festsetzen, womit besonders bei Trägern von Zahnersatz Probleme auftreten. Ein weiterer Nachteil partikelhaltiger Suspensionen ist es, daß die Guar-Partikel entweder an der Oberfläche schwimmen oder rasch sedimentieren, so daß dem Patienten weniger als eine Minute Zeit bleibt, um eine einigermaßen homogene Suspension nach raschem Umrühren zu trinken. Die Folge ist eine Verweigerung des Medikamentes oder jedenfalls dessen unregelmäßiger Gebrauch.

Aufgabe der Erfindung ist es somit, Guar-Mehl in eine derartige Form zu bringen, die es insbesondere auch älteren und wenig belastbaren Patienten erlaubt, mehrmals täglich Mengen in der Größe von etwa 3 bis 5 g einzunehmen. Dabei sollten weder große und schwer zu schluckende Arzneiformen, wie beispielsweise Kapseln, gewählt werden, noch sollte das Guar-Mehl in zu großen Flüssigkeitsmengen getrunken werden müssen, Klumpen bilden oder unangenehme Geschmackseigenschaften aufweisen oder aufgrund seiner Konsistenz für den Patienten störend sein. Andererseits sollen die geschilderten vorteilhaften Eigenschaften des Guar-Mehls erhalten bleiben.

Gegenstand der Erfindung ist daher ein oral zu verabfolgendes Arzneimittel, welches sich dadurch auszeichnet, daß es einen Gehalt an physikalisch und chemisch unverändertem losen Guarmehl, dessen Korngröße zu 90-100 Gew.-% im Bereich von 50 - 1500 µm liegt, aufweist.

Weiterhin sind Gegenstände der Erfindung oral zu verabfolgende Arzneimittel zur Verzögerung der Glucoseabsorption und zur Senkung des Blutcholesterinspiegels sowie zur Verringerung eines vorzeitigen Hungergefühls und zur Regulierung der intestinalen Peristaltik beim Menschen, die sich jeweils durch einen Gehalt an physikalisch und chemisch unverändertem losen Guarmehl, dessen Korngröße zu 90-100 Gew.-% im Bereich von 50 - 1500 µm liegt, auszeichnen.

Ein weiterer Gegenstand der Erfindung ist ein oral zu verabfolgendes Kontrastmittel, welches durch eine Beimengung an physikalisch und chemisch unverändertem Guar-Mehl, dessen Korngröße zu 90-100 Gew.-% im Bereich von 50-1500 µm liegt, gekennzeichnet ist.

Die Erfindung stellt eine überraschend einfache Lösung des seit Jahren bearbeiteten Problems dar. Im Ergebnis wird ohne Verwendung von Zusatzstoffen ein reines Guar-Mehl eingesetzt, das mit wenig Wasser bequem, risikolos und angenehm eingenommen werden kann.

Da keine besonderen Vorkehrungen zur Verminderung oder Verzögerung der Quellfähigkeit von Guar notwendig sind, besteht auch keinerlei Risiko, daß das Guar nach der Einnahme nicht vollständig durchquillt.

Grundlage der Erfindung ist die Beobachtung, daß der Feinanteil (Körner <<100 µm) der gebräuchlichen Guar-Mehle, auch wenn ihr mengenmäßiger Anteil sehr gering ist, für das äußerst rasche Gelieren der Aufschwemmungen der üblichen Guar-Mehle verantwortlich ist. Dadurch werden beispielsweise 4g gewöhnliches Guar-Mehl - eingebracht in 150 bis 200 ml Wasser - binnen 10 bis 30 Sekunden untrinkbar viskos. Ein weiterer wesentlicher Nachteil der gebräuchlichen Guar-Mehle ist die fast unvermeidliche Bildung von Klumpen unverquollenen Mehls, eingeschlossen in eine undurchlässige Gallerte-Schicht. Diese Klumpen erschweren zusätzlich die Einnahme der ohnehin hochviskosen Gallerte. Siebt man den Anteil an Feinpartikeln ab, so daß das verbleibende Guar-Mehl beispielsweise zu >95% aus Partikeln der Größe 100 µm bis 500 µm besteht, erhält man nach Einrühren von 4g Mehl in 100 bis 200 ml Wasser eine homogene, hinreichend dünnflüssige Suspension. Das entsprechende ausgewählte Mehl ist ausgezeichnet benetzbar, es sinkt sofort in der Flüssigkeit unter, läßt sich leicht umrühren, bildet dabei keine Klumpen und enthält keine im Mund spürbaren Partikel. Es bleibt bei Zimmertemperatur für mindestens fünf Minuten dünnflüssig genug, um getrunken zu werden. In Wasser angerührt ist das beschriebene Mehl weitgehend geschmacksneutral, in Fruchtsäften oder anderen Flüssigkeiten nimmt es deren Geschmack unverfälscht an.

Ein erfindungsgemäß hergestelltes Mittel enthält beispielsweise chemisch unverändertes Guar-Mehl ohne jegliche Zusätze, charakterisiert durch einen Anteil von >90%, bevorzugt >98% Guar-Partikeln mit einem Korndurchmesser von 50 µm bis 1500 µm, vorzugsweise 100 bis 500 µm. Die Dosierung kann beispielsweise über einen Meßlöffel aus einem Vorratsbehälter oder genauer und hygienisch einwandfreier durch Abpackung der Einzeldosis in Einzelbehältnisse erfolgen. Die Einzeldosis beträgt ca. 3 bis 6 g, vorzugsweise 4g reines Guar-Mehl. Es werden 2 bis 5 Einzeldosen/Tag eingenommen. Das Mehl wird mit einem entsprechenden Volumen wässriger Flüssigkeit, vorzugsweise 100 bis 200 ml/je 4g Mehl gemischt und innerhalb von ca. 0 bis 20, vorzugsweise 0 bis 5 Minuten nach dem vollständigen Mischen getrunken.

Dem Mehl selbst können Geschmacks- und Aromastoffe, Mittel zum Aufschäumen, wie $NaHCO_3$ sowie Zitronensäure, Vitamine, Mineralstoffe und insbesondere Arzneistoffe zugesetzt werden, deren Resorption zeitlich verzögert werden soll. Letztere Variante ist besonders dann wertvoll, wenn die Erkrankungen des Patienten eine gleichzeitige Behandlung mit Guar und einem Arzneistoff erfordern, der normalerweise entweder zu hohe Blutspiegel erreicht (z.B. orale Antidiabetika) oder eine zu kurze Halbwertszeit aufweist (z.B. Lipidsenker).

Ferner kann das erfindungsgemäß eingesetzte Guar-Mehl oral verabfolgt werden zur Senkung des Blutcholesterinspiegels oder zur Regulierung der Darmperistaltik.

Bei diagnostischen Untersuchungen des Magens oder Darms kann eine gleichmäßige Füllung des Lumens oder ein Wandbeschlag erwünscht sein. Auch in diesem Fall ist die Anwendung der beschriebenen Guar-Zubereitung von Nutzen, insbesondere wegen ihrer guten Dispergierbarkeit und des angenehmen Geschmacks. Die Mischbarkeit mit Kontrastmitteln verschiedener Art ist einwandfrei gegeben. Im Magen und Darm werden die erwünschten hohen Viskositäten durch vollständige Quellung auch der größeren Guar-Partikel erreicht. Im Falle osmotisch aktiver Kontrastmittel kann der Zusatz von ausreichenden Mengen Guar deren Diarrhoe auslösende Wirkung deutlich vermindern.

Der Zusatz der genannten Stoffe, insbesondere auch der geschmacksbestimmenden Stoffe, kann sehr gut durch die Wahl der Flüssigkeit (z.B. Fruchtsäfte), in der das Guar-Mehl suspendiert wird, erfolgen. Auf diese Weise bleibt die Wahl der Geschmacksrichtung dem Patienten überlassen.

Bevorzugt sind wässrige Suspensionen, in denen der Gehalt des Guar-Mehls mindestens 2 Gew.-% beträgt. Die erfindungsgemäß erforderlichen Korngrößenfraktionen werden durch übliche Trennvorgänge, beispielsweise durch Sieben, aus im Handel befindlichen Guar-Mehlen erhalten.

## Beispiel 1

4g Guar-Mehl, Korngröße >98% >100 $\mu$m <500 $\mu$m, abgepackt in Beuteln. Der Beutelinhalt wird in 150 ml Wasser bei Raumtemperatur suspendiert und bald danach getrunken.

## Beispiel 2

Ca. 6g Guar-Mehl, Korngröße 95 Gew.-% >200 $\mu$m <1000 $\mu$m, werden mit einem Meßlöffel aus einem Vorratsbehälter entnommen und in 200 ml gasfreier Orangenlimonade suspendiert und binnen fünf Minuten getrunken.

## Beispiel 3

4g Guar-Mehl, Korngröße 90 Gew.-% >50 <500 $\mu$m, werden in Aluminiumfolie einzeln abgepackt. Der gesamte Inhalt einer Einzelpackung wird zu 150 ml kaltem (ca. 10°C) Wasser hinzugegeben und rasch getrunken.

## Beispiel 4

4g Guar-Mehl, Korngröße 98 Gew.-% >100 $\mu$m <1500 $\mu$m, werden in 150 ml frisch gepreßtem kalten Fruchtsaft (Orange, Apfel) suspendiert und bald darauf getrunken.

## Beispiel 5

3g Guar-Mehl, Korngröße >98 Gew.-% >100 $\mu$m <500 $\mu$m, werden gleichmäßig mit 50 mg Vitamin C, 20 mg $MgCO_3$ und 20 mg $NaHCO_3$ gemischt und in einen Beutel verpackt. Der gesamte Beutelinhalt wird in 100 ml kaltes Wasser eingerührt und bald getrunken.

## Beispiel 6

4g Guar-Mehl, Korngröße >98 Gew.-% >100 $\mu$m <500 $\mu$m, werden mit 3,5 mg Glibenclamid als antidiabetisch wirkendem Mittel gleichmäßig vermengt und in einen Beutel abgefüllt. Der gesamte Beutelinhalt wird in 150 ml Wasser suspendiert und bald getrunken.

## Beispiel 7

4

4g Guar-Mehl, Korngröße >98 Gew.-% >100 um <500 μm, werden mit 50 mg Magnesiumpyridoxalphosphatglutamat (I) und 5 mg MgCO$_3$ (II) gleichmäßig vermischt, indem die 1%-ige (I) bzw. 0,1%-ige (II) Lösung von I und II in 80%-igem Ethanol auf das Guar-Mehl aufgezogen und der Alkohol unter vorsichtigem Rühren bei 40°C abgezogen wird. Der Rückstand wird unter vermindertem Druck auf einen Wassergehalt von <5% getrocknet. Die gesamte Zubereitung wird in Aluminiumbeutel verpackt. Der Inhalt der Beutel ist in 150 ml Wasser einzurühren und innerhalb von ca. 5 Minuten zu trinken.

Beispiel 8

8g Guar-Mehl,Korngröße 95 Gew.-% >50 <250 μm, werden mit einer 10g Jod enthaltenden Menge Natrium-Methylglucaminamidotrizoat (10:66) vermischt und in aluminiumbeschichteten Beuteln abgepackt. Unmittelbar vor Gebrauch wird der Beutelinhalt in 1 Liter Wasser eingerührt und langsam getrunken. Die Untersuchung erfolgt mit der Computertomographie kurz nach oder maximal 16 Stunden nach Aufnahme der Zubereitung. Gegebenenfalls können kurz vor der Untersuchung nochmals 500 ml bis 1000 ml eingenommen werden.

Vergleichsversuche

In den Vergleich einbezogen wurden die für die Anwendung als Arzneimittel zugelassenen Präparate GLUCOTARD® (unter Zusatz von Kieselgel zu Tabletten verpreßtes Guar-Mehl) der Firma Boehringer Mannheim, und GUAREM® (Guar-Granulat mit Hilfsstoff-Zusatz) der Fa. Lääketehdas Remeda, Finnland, die gewöhnlichen Guarmehle Müggenburg, DIAGUAR-63 und DIAGUAR-250 sowie ein Präparat (GU-052) entsprechend Beispiel 1 der vorliegenden Erfindung.

Als Bewertungskriterien galten "gute Einnehmbarkeit" sowie "vollständige Quellbarkeit". Die Zielkriterien für die Einnehmbarkeit waren:
- keine Erhöhung der Dosis durch Hilfsstoffe
- keine Tabletten oder Partikel
- für Suspensionen eine gleichmäßige Verteilung in Wasser, ohne daß das Material an der Oberfläche verharrt oder rasch sedimentiert oder gar verklumpt
- ein neutraler Geschmack
- Trinkbarkeit, die als Fließfähigkeit unter den in Tabelle 1 definierten Bedingungen objektiviert wurde.

Die Quellfähigkeit wurde anhand der Viskosität einer 1%-igen Zubereitung in Wasser nach begrenzter Quellzeit beurteilt.

Methode zur Ermittlung der Trinkfähigkeit

Das Guar-Mehl wird zunächst in dem jeweiligen Volumen Wasser bei 18°C gerührt. Sodann wird die möglichst homogene Suspension nach etwa 30 Sekunden in einen 110 ml fassenden zylindrischen Vorratsbehälter mit einem Durchmesser von etwa 15 mm und mit einem angesetzten zylindrischen Rohr von 20 mm Länge und einer Öffnung von 5 mm im Durchmesser eingegeben. Die Auslauföffnung wird nach genau einer Minute, nachdem das Guar-Material mit dem Wasser in Berührung gekommen ist, freigegeben. Anschließend wird die Zeit für den Auslauf von 10 und 30 ml gemessen. In einem zweiten Versuch wird die Auslauföffnung erst nach fünf Minuten Quellzeit freigegeben.

Bewertung: Solange die Guar-Suspension aus dem Behälter ausläuft, wird sie als trinkfähig angesehen.

**TAB. 1**
Kriterien zur Bewertung von oral einzunehmenden Guar-Präparaten und vergleichende Prüfung unterschiedlicher Zubereitungen

| | Beispiel 1 GU-052 | GLUCOTARD® | GUAREM® | GUAR Müggenburg | DIAGUAR -63 | DIAGUAR -250 |
|---|---|---|---|---|---|---|
| Menge (g) | 4,0 | 5,0 | 4,75 | 4,0 | 4,0 | 4,0 |
| davon Hilfsstoffe (g) | - | 1,133 | 0,25 | - | - | - |
| Tabletten | - | ja | - | - | - | - |
| **Suspensionen** | | | | | | |
| Material schwimmt | - | - | - | ja | ja | - |
| Material sedimentiert | sehr langsam | sehr rasch | sehr rasch | - | - | - |
| bildet Klumpen | - | - | - | ja | stark | sehr wenig |
| störende Partikel im | - | ja | sehr störend | ja, Klumpen | ja, Klumpen | - |
| Geschmack | neutral | leicht sauer | neutral | neutral | neutral | neutral |
| **Fließfähigkeit** | | | | | | |
| nach 1 min 18°C (30 ml in sec.) | 3,4 | * | 2,7 | 12 | 17 | 26 |
| nach 5 min 18°C (10 ml in sec.) | 106 | * | 1,0 | nicht fließfähig | nicht fließfähig | nicht fließfähig |
| **Quellfähigkeit** Viskosität 1%-ig, nach 2 h, 20°C (cP) | 5350 | 650 | 1360 | * | 5500 | 5020 |

"-" bedeutet "null" oder "nein"     "*" bedeutet "keine Messung"

## Ergebnis

Das erfindungsgemäße Präparat GU-052 entspricht den Zielkriterien am besten. Es enthält keine Hilfsstoffe und keine spürbaren Partikel, ist klumpenfrei in Wasser suspendierbar, schwimmt und sedimen-

6

EP 0 241 710 B1

tiert nicht, ist geschmacklich neutral und bleibt, suspendiert in 200 ml (=einer Tasse) Wasser, für mindestens 5 Minuten leicht trinkbar.

Das erfindungsgemäße Präparat GU-052 ist im Gegensatz zu den Präparaten GLUCOTARD® und GUAREM® voll quellfähig und erfüllt damit eine essentielle Anforderung für eine gute Wirksamkeit. Dies betrifft insbesondere die Anwendungsmöglichkeiten, bei denen es um die Verzögerung einer unerwünscht raschen Resorption geht (z.B. Glucose beim Diabetiker).

**Patentansprüche**

1.  Oral zu verabfolgendes Arzneimittel, **gekennzeichnet durch** einen Gehalt an physikalisch und chemisch unverändertem losen Guarmehl, dessen Korngröße zu 90-100 Gew.-% im Bereich von 50-1500 $\mu$m liegt.

2.  Oral zu verabfolgendes Arzneimittel zur Verzögerung der Glucoseabsorption und zur Senkung des Blutcholesterinspiegels, **gekennzeichnet durch** einen Gehalt an physikalisch und chemisch unverändertem losen Guarmehl, dessen Korngröße zu 90-100 Gew.-% im Bereich von 50-1500 $\mu$m liegt.

3.  Oral zu verabfolgendes Arzneimittel zur Verringerung eines vorzeitigen Hungergefühls und zur Regulierung der intestinalen Peristaltik beim Menschen, **gekennzeichnet durch** einen Gehalt an physikalisch und chemisch unverändertem losen Guarmehl, dessen Korngröße zu 90-100 Gew.-% im Bereich von 50-1500 $\mu$m liegt.

4.  Oral zu verabfolgendes Kontrastmittel, **gekennzeichnet durch** eine Beimengung an physikalisch und chemisch unverändertem Guarmehl, dessen Korngröße zu 90-100 Gew.-% im Bereich von 50-1500 $\mu$m liegt.

**Claims**

1.  Medicament to be administered orally, characterised in that it contains physically and chemically unmodified loose guar flour whose particle size is up to 90-100 % by weight in the range from 50-1500 $\mu$m.

2.  Medicament to be administered orally for delaying the absorption of glucose and for lowering the blood cholesterol level, characterised in that it contains physically and chemically unmodified loose guar flour whose particle size is up to 90-100 % by weight in the range from 50-1500 $\mu$m.

3.  Medicament to be administered orally for reducing a premature feeling of hunger and for regulating intestinal peristalsis in humans, characterised in that it contains physically and chemically unmodified loose guar flour whose particle size is up to 90-100 % by weight in the range from 50-1500 $\mu$m.

4.  Contrast agent to be administered orally, characterised in that physically and chemically unmodified guar flour whose particle size is up to 90-100 % by weight in the range from 50-1500 $\mu$m is admixed.

**Revendications**

1.  Médicament pour administration orale, caractérisé en ce qu'il contient de la farine de guar libre, non modifiée physiquement ou chimiquement, dont la dimension des grains est comprise, pour 90 à 100 % de son poids poids, entre 50 et 1500 $\mu$m.

2.  Médicament pour administration orale, pour retarder l'absorption du glucose et abaisser le taux des lipides du sérum, caractérisé en ce qu'il contient de la farine de guar libre, non modifiée physiquement ou chimiquement, dont la dimension des grains est comprise, pour 90 à 100 % de son poids poids, entre 50 et 1500 $\mu$m.

3.  Médicament pour administration orale, pour atténuer une sensation de faim prématurée et pour réguler le péristaltisme intestinal chez l'homme, caractérisé en ce qu'il contient de la farine de guar libre, non modifiée physiquement ou chimiquement, dont la dimension des grains est comprise, pour 90 à 100 % de son poids poids, entre 50 et 1500 $\mu$m.

7

4. Agent de contraste pour administration orale, caractérisé par l'addition de 0,5 à 2,5 % en poids de farine de guar, non modifiée physiquement ou chimiquement, dont la dimension des grains est comprise, pour 90 à 100 % de son poids, entre 50 et 1500 $\mu$m.